(19) Europäisches Patentamt
European Patent Office
Office européen des brevets

(11) Veröffentlichungsnummer: **0 532 948 A1**

(12) **EUROPÄISCHE PATENTANMELDUNG**

(21) Anmeldenummer: **92114571.0**

(22) Anmeldetag: **27.08.92**

(51) Int. Cl.⁵: **C07D 239/60**, C07D 239/52, A01N 43/54

(30) Priorität: **09.09.91 DE 4129876**

(43) Veröffentlichungstag der Anmeldung: **24.03.93 Patentblatt 93/12**

(84) Benannte Vertragsstaaten: **BE CH DE ES FR GB IT LI NL**

(71) Anmelder: **BAYER AG**

**W-5090 Leverkusen 1 Bayerwerk(DE)**

(72) Erfinder: **Drewes, Mark-Wilhelm, Dr.**
**Talstrasse 54**
**W-4018 Langenfeld(DE)**
Erfinder: **Müller, Peter, Dr.**
**Talstrasse 54**
**W-4018 Langenfeld(DE)**
Erfinder: **Santel, Hans-Joachim, Dr.**
**Grünstrasse 9a**
**W-5090 Leverkusen 1(DE)**
Erfinder: **Lürssen, Klaus, Dr.**
**August-Kierspel-Strasse 145**
**W-5060 Bergisch Gladbach 2(DE)**
Erfinder: **Schmidt, Robert, Dr.**
**Im Waldwinkel 110**
**W-5060 Bergisch Gladbach 2(DE)**

(54) **Substituierte Cycloalkene als Herbizide.**

(57) Die Erfindung betrifft neue substituierte Cycloalkene der allgemeinen Formel (I)

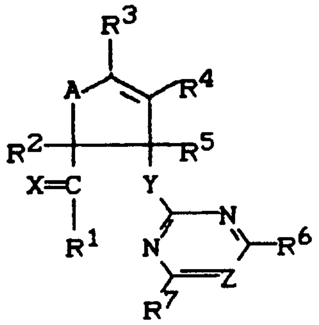

(I)

in welcher
A,$R^1$,$R^2$,$R^3$,$R^4$,$R^5$,$R^6$,$R^7$,X,Y und Z die in der Beschreibung angegebenen Bedeutungen haben,
ein Verfahren zu ihrer Herstellung sowie ihre Verwendung als Herbizide.

EP 0 532 948 A1

Die Erfindung betrifft neue substituierte Cycloalkene, ein Verfahren zu ihrer Herstellung und ihre Verwendung als Herbizide.

Substituierte Cycloalkene haben bisher keine Bedeutung als Herbizide erlangt.

Es wurden nun neue substituierte Cycloalkene der allgemeinen Formel (I) gefunden,

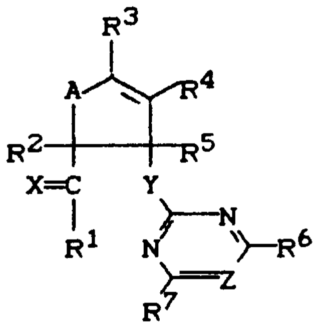

(I)

in welcher

| | |
|---|---|
| A | für geradkettiges oder verzweigtes, gegebenenfalls substituiertes Alkandiyl steht, |
| $R^1$ | für Wasserstoff, Hydroxy, Amino oder für einen jeweils gegebenenfalls substituierten Rest der Reihe Alkyl, Alkoxy, Alkylthio, Alkylamino oder Dialkylamino steht, |
| $R^2$, $R^3$, $R^4$ und $R^5$ | gleich oder verschieden sind und für Wasserstoff, Hydroxy, Amino, Cyano, Halogen, Alkyl, Halogenalkyl, Alkoxy, Alkylthio, Aryl, Alkoxycarbonyl oder Alkoxycarbonylalkyl stehen, |
| $R^6$ und $R^7$ | gleich oder verschieden sind und für Wasserstoff, Halogen oder für einen jeweils gegebenenfalls substituierten Rest der Reihe Alkyl, Alkoxy, Alkylthio, Alkylamino oder Dialkylamino stehen, |
| X | für Sauerstoff, Schwefel oder eine der nachstehenden Gruppierungen steht |
| N-$R^8$ | oder |

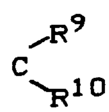

wobei

| | |
|---|---|
| $R^8$ | für Wasserstoff, Hydroxy, Amino oder für einen jeweils gegebenenfalls substituierten Rest der Reihe Alkyl, Alkenyl, Alkinyl, Alkoxy, Alkenyloxy, Alkoxycarbonylalkoxy, Alkylamino, Dialkylamino, Alkylcarbonylamino, Alkoxycarbonylamino, Alkylsulfonylamino, Aryl, Aralkyl, Aryloxy, Aralkyloxy, Arylamino, Aralkylamino, N-Alkyl-N-arylamino, Hetarylamino, Hetarylcarbonylamino, Arylcarbonylamino oder Arylsulfonylamino steht, |
| $R^9$ | für Wasserstoff, Halogen, Cyano, Carboxyl, Alkoxycarbonyl, Alkylcarbonylamino oder Dialkoxyphosphoryl steht und |
| $R^{10}$ | für Formyl, Cyano, Carboxyl, Hydroxymethyl, Carbamoyl oder für einen jeweils gegebenenfalls substituierten Rest der Reihe Alkoxycarbonyl, Cycloalkoxycarbonyl, Alkylthiocarbonyl, Alkylaminocarbonyl, Cycloalkylaminocarbonyl, Dialkylaminocarbonyl, Alkylaminocarbonylalkoxycarbonyl, Dialkylaminocarbonylalkoxycarbonyl, Arylaminocarbonylalkoxycarbonyl, N-AlkylN-arylaminocarbonylalkoxycarbonyl, Pyrrolidinylcarbonyl, Piperidinylcarbonyl, Morpholinylcarbonyl, Piperazinylcarbonyl, Aryloxycarbonyl, Aralkyloxycarbonyl, Heterocyclylalkoxycarbonyl, Arylthiocarbonyl, Aralkylthiocarbonyl, Arylaminocarbonyl, Aralkylaminocarbonyl, N-Alkyl-N-arylaminocarbonyl, Arylhydrazinocarbonyl, Alkylhydrazinocarbonyl, Phthalimidoxycarbonyl oder $R^{10}$ zusammen mit $R^9$ für die Gruppierung $-CO-O-(CH_2)_n-$ steht, wobei n für die Zahlen 1 bis 4 steht, |
| Y | für Sauerstoff, Schwefel, Imino (NH) oder Alkylimino (N-Alkyl) steht, und |

Z für Stickstoff oder die Gruppierung C-$R^{11}$ steht, worin $R^{11}$ für Wasserstoff, Halogen, Alkyl oder Alkoxy steht.

Man erhält die neuen substituierten Cyclohexene der allgemeinen Formel (I), wenn man Cycloalkene der allgemeinen Formel (II)

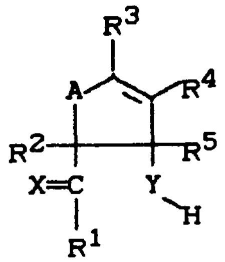

(II)

in welcher
A, $R^1$, $R^2$, $R^3$, $R^4$, $R^5$, X und Y die oben angegebenen Bedeutungen haben,
mit Azinen der allgemeinen Formel (III)

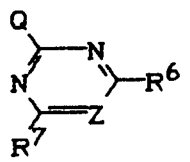

(III)

in welcher
$R^6$, $R^7$ und Z die oben angegebenen Bedeutungen haben und
Q für eine nucleofuge Abgangsgruppe steht,
gegebenenfalls in Gegenwart eines Säureakzeptors und gegebenenfalls in Gegenwart eines Verdünnungsmittels umsetzt.

Die neuen substituierten Cycloalkene zeichnen sich durch starke Herbizidwirkung aus.

Die Erfindung betrifft vorzugsweise Verbindungen der Formel (I), in welcher

A für geradkettiges oder verzweigtes Alkandiyl mit 1 bis 5 Kohlenstoffatomen steht, welches gegebenenfalls durch Hydroxy, Amino, Halogen, Phenyl oder $C_1$-$C_5$-Alkoxy-carbonyl substituiert ist,

$R^1$ für Wasserstoff, Hydroxy, Amino oder für einen jeweils gegebenenfalls durch Halogen, $C_1$-$C_5$-Alkoxy oder Oxiranyl substituierten Rest der Reihe $C_1$-$C_5$-Alkyl, $C_1$-$C_5$-Alkoxy, $C_1$-$C_5$-Alkylthio, $C_1$-$C_5$-Alkylamino oder Di-($C_1$-$C_5$-alkyl)-amino steht,

$R^2$, $R^3$, $R^4$ und $R^5$ gleich oder verschieden sind und für Wasserstoff, Hydroxy, Amino, Cyano, Halogen, $C_1$-$C_5$-Alkyl, $C_1$-$C_5$-Alkoxy, $C_1$-$C_5$-Alkylthio, Phenyl oder $C_1$-$C_5$-Alkoxycarbonyl stehen,

$R^6$ und $R^7$ gleich oder verschieden sind und für Wasserstoff, Halogen oder für einen jeweils gegebenenfalls durch Halogen oder $C_1$-$C_3$-Alkoxy substituierten Rest der Reihe $C_1$-$C_4$-Alkyl, $C_1$-$C_4$-Alkoxy, $C_1$-$C_4$-Alkylthio, $C_1$-$C_4$-Alkylamino oder Di-($C_1$-$C_4$-alkyl)-amino stehen,

X für Sauerstoff, Schweferl oder eine der nachstehenden Gruppierungen steht

N-$R^8$ oder

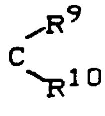

wobei

$R^8$ für Wasserstoff, Hydroxy, Amino oder für einen jeweils gegebenenfalls durch Halogen substituierten Rest der Reihe $C_1$-$C_6$-Alkyl, $C_3$-$C_6$-Alkenyl, $C_3$-$C_6$-Alkinyl, $C_1$-$C_6$-Alkoxy, $C_3$-$C_6$-Alkenyloxy, $C_1$-$C_4$-Alkoxy-carbonyl-$C_1$-$C_2$-alkoxy, $C_1$-$C_6$-Alky-

lamino, Di-($C_1$-$C_2$-alkyl)-amino, $C_1$-$C_6$-Alkylcarbonylamino, $C_1$-$C_6$-Alkoxy-carbonylamino, $C_1$-$C_6$-Alkylsulfonylamino, oder für jeweils gegebenenfalls durch Nitro, Amino, Cyano, Carboxyl, Halogen, $C_1$-$C_4$-Alkyl, $C_1$-$C_2$-Halogenalkyl, $C_1$-$C_4$-Alkoxy, $C_1$-$C_2$-Halogenalkoxy, $C_1$-$C_4$-Alkylthio, $C_1$-$C_2$-Halogenalkylthio, $C_1$-$C_4$-Alkoxy-carbonyl und/ oder Di-($C_1$-$C_2$-alkyl)-amino substituierten Rest der Reihe Phenyl, Phenyl-$C_1$-$C_4$-alkyl, Phenoxy, Phenyl-$C_1$-$C_4$-alkoxy, Phenylamino, Phenyl-$C_1$-$C_4$-alkylamino, N-($C_1$-$C_4$-alkyl)-N-phenylamino, Pyridylamino, Pyrimidylamino, Pyridylcarbonylamino, Phenylcarbonylamino, Furylcarbonylamino, Thienylcarbonylamino oder Phenylsulfonylamino steht,

$R^9$ für Wasserstoff, Halogen, Cyano, Carboxyl, $C_1$-$C_6$-Alkoxycarbonyl, $C_1$-$C_6$-Alkylcarbonylamino oder Di-($C_1$-$C_4$-alkoxy)-phosphoryl steht und

$R^{10}$ für Formyl, Cyano, Carboxyl, Hydroxymethyl, Carbamoyl oder für einen jeweils gegebenenfalls durch Halogen, Carboxyl oder $C_1$-$C_4$-Alkoxycarbonyl substituierten Rest der Reihe $C_1$-$C_6$-Alkoxycarbonyl, $C_5$-$C_6$-Cycloalkyloxycarbonyl, $C_1$-$C_6$-Alkylthio-carbonyl, $C_5$-$C_6$-Cycloalkylaminocarbonyl, Di-($C_1$-$C_2$-alkyl)-aminocarbonyl, $C_1$-$C_4$-Alkylaminocarbonyl-$C_1$-$C_4$-alkoxycarbonyl, Di-($C_1$-$C_2$-alkyl)-aminocarbonyl-$C_1$-$C_4$-alkoxycarbonyl, Phenylaminocarbonyl-$C_1$-$C_4$-alkoxycarbonyl, N-Methyl-N-phenylaminocarbonyl-$C_1$-$C_4$-alkoxy-carbonyl, oder für einen jeweils gegebenenfalls durch Methyl und/oder Ethyl substituierten Rest der Reihe Pyrrolidinylcarbonyl, Piperidinylcarbonyl, Morpholinylcarbonyl oder Piperazinylcarbonyl, oder für einen jeweils gegebenenfalls durch Nitro, Amino, Cyano, Carboxyl, Halogen, $C_1$-$C_4$-Alkyl, $C_1$-$C_2$-Halogenalkyl, $C_1$-$C_4$-Alkoxy, $C_1$-$C_2$-Halogenalkoxy, $C_1$-$C_4$-Alkylthio, $C_1$-$C_2$-Halogenalkylthio, $C_1$-$C_4$-Alkoxy-carbonyl und/oder Di-($C_1$-$C_2$-alkyl)-amino substituierten Rest der ReihePhenoxycarbonyl, Phenyl-$C_1$-$C_4$-alkoxycarbonyl, Furylmethoxycarbonyl, Thienylmethoxycarbonyl, Phenylthiocarbonyl, Phenyl-$C_1$-$C_4$-alkylthio-carbonyl, Phenylaminocarbonyl, Phenyl-$C_1$-$C_4$-alkylaminocarbonyl,N-($C_1$-$C_4$-Alkyl)-N-phenylaminocarbonyl oder Phenylhydrazinocarbonyl, $C_1$-$C_4$-Alkylhydrazinocarbonyl, für Phthalimidoxycarbonyl oder zusammen mit $R^9$ für die Gruppierung $-CO-O-(CH_2)_n-$ steht, wobei n für die Zahlen 1 bis 4 steht,

Y für Sauerstoff, Schwefel, Imino (NH) oder Methylimino ($NCH_3$) steht und

Z für Stickstoff oder die Gruppierung C-$R^{11}$ steht, worin

$R^{11}$ für Wasserstoff, Fluor, Chlor, Methyl oder Methoxy steht.

Die in der Definition der erfindungsgemäßen Verbindungen aufgeführten aliphatischen Kohlenwasserstoffreste (z.B. Alkyl, Alkenyl, Alkinyl), auch in Kombination mit Heteroatomen (z.B. in Alkoxy, Alkylthio, Alkylamino) oder in Zusammensetzungen wie z.B. Halogenalkyl, Halogenalkoxy, sind jeweils geradkettig oder verzweigt.

Halogen steht im allgemeinen für Fluor, Chlor, Brom oder Jod, vorzugsweise für Fluor, Chlor oder Brom, insbesondere für Fluor oder Chlor.

Die Erfindung betrifft insbesondere Verbindungen der Formel (I), in welcher

A für Methan-1,1-diyl (Methylen, $-CH_2-$), Ethan-1,1-diyl

(Ethyliden, 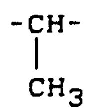 ),

Ethan-1,2-diyl (Dimethylen, $-CH_2CH_2-$), Propan-1,1-diyl

(Propyliden, 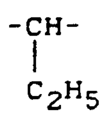 ),

Propan-1,2-diyl

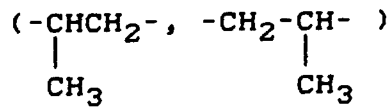

2-Methyl-propan-1,2-diyl

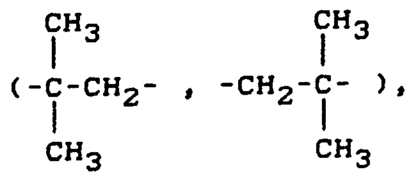

Propan-1,3-diyl (Trimethylen, $-CH_2CH_2CH_2-$), Butan-1,2-diyl

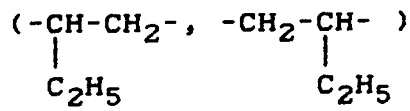

oder Butan-2,3-diyl

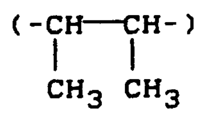

steht,

$R^1$ für Wasserstoff, Hydroxy, Amino oder für einen jeweils gegebenenfalls durch Fluor, Chlor, Methoxy oder Ethoxy substituierten Rest der Reihe Methyl, Ethyl, Propyl, Isopropyl, Methoxy, Oxiranylmethoxy, Ethoxy, Propoxy, Isopropoxy, Methylamino, Ethylamino, Propylamino, Isopropylamino, Dimethylamino oder Diethylamino steht,

$R^2$, $R^3$, $R^4$ und $R^5$ gleich oder verschieden sind und für Wasserstoff, Hydroxy, Amino, Fluor, Chlor, Brom, Methyl, Ethyl, Propyl, Isopropyl, Phenyl, Methoxycarbonyl oder Ethoxycarbonyl stehen,

$R^6$ und $R^7$ gleich oder verschieden sind und für Wasserstoff, Chlor, Methyl, Ethyl, Trifluormethyl, Methoxymethyl, Methoxy, Ethoxy, Difluormethoxy, Methylthio, Methylamino, Ethylamino oder Dimethylamino stehen,

X für Sauerstoff, Schwefel oder eine der nachstehenden Gruppierungen steht

$N-R^8$ oder

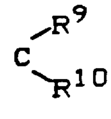

wobei

$R^8$ für Wasserstoff, Hydroxy, Amino, für Methyl, Ethyl, n-Propyl, Isopropyl, n-Butyl, Isobutyl, sec-Butyl, tert-Butyl, Allyl, Propargyl, Methoxy, Ethoxy, n-Propoxy, Isopropoxy, n-Butoxy, Isobutoxy, sec-Butoxy, Allyloxy, Methoxycarbonylmethoxy, Ethoxycarbonylmethoxy, Methoxycarbonylethoxy, Ethoxycarbonylethoxy, Methylamino, Ethylamino, n-Propylamino, Isopropylamino, n-Butylamino, Isobutylamino, sec-Butylamino, tert-Butylamino, Dimethylamino, Acetylamino, Propionylamino, Methoxycarbonylamino, Ethoxycarbonylamino, Methylsulfonylamino, Ethylsulfonylamino, für jeweils gegebenenfalls durch Nitro, Cyano, Fluor, Chlor, Brom, Methyl,

Trifluormethyl, Methoxy, Trifluormethoxy, Methylthio oder Trifluormethylthio substituiertes Phenyl, Benzyl, Phenoxy, Benzyloxy, Phenylamino, Benzylamino, N-Methyl-N-phenylamino, Pyridylamino, Pyrimidylamino, Pyridylcarbonylamino, Phenylcarbonylamino, Furylcarbonylamino, Thienylcarbonylamino oder Phenylsulfonylamino steht,

$R^9$ für Wasserstoff, Fluor, Chlor, Cyano, Carboxyl, $C_1$-$C_4$-Alkoxycarbonyl, $C_1$-$C_4$-Alkylcarbonylamino, Dimethoxyphosphoryl oder Diethoxyphosphoryl steht und

$R^{10}$ für Formyl, Cyano, Carboxyl, Hydroxymethyl, Carbamoyl, für einen jeweils gegebenenfalls durch Fluor, Chlor, Carboxy oder $C_1$-$C_4$-Alkoxycarbonyl substituierten Rest der Reihe $C_1$-$C_4$-Alkoxycarbonyl, $C_5$-$C_6$-Cycloalkyloxycarbonyl, $C_1$-$C_4$-Alkylthiocarbonyl, $C_1$-$C_4$-Alkylaminocarbonyl, $C_5$-$C_6$-Cycloalkylaminocarbonyl, Dimethylaminocarbonyl, $C_1$-$C_4$-Alkylaminocarbonyl$C_1$-$C_4$-alkoxycarbonyl, Dimethylaminocarbonyl-$C_1$-$C_4$-alkoxycarbonyl, N-Methyl-N-phenylaminocarbonyl-$C_1$-$C_4$-alkoxycarbonyl, oder für einen jeweils gegebenenfalls durch Methyl und/oder Ethyl substituierten Rest der Reihe Pyrrolidinylcarbonyl, Piperidinylcarbonyl, Morpholinylcarbonyl oder Piperazinylcarbonyl,
oder für jeweils gegebenenfalls durch Nitro, Cyano, Fluor, Chlor, Brom, Methyl, Trifluormethyl, Methoxy, Trifluormethoxy, Methylthio oder Trifluormethylthio substituierten Rest der Reihe Phenoxycarbonyl, Benzyloxycarbonyl, Phenylthiocarbonyl, Benzylthiocarbonyl, Phenylaminocarbonyl, Benzylaminocarbonyl, N-Methyl-N-phenylaminocarbonyl, Phenylhydrazinocarbonyl, für Phthalimidoxycarbonyl oder zusammen mit $R^9$ für die Gruppierungen $-CO-O-CH_2CH_2-$,$-COO-CH_2CH_2CH_2-$,

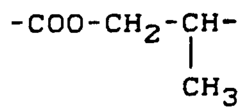

oder

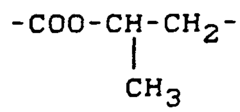

steht,

Y für Sauerstoff oder Imino (NH) steht und

Z für Stickstoff oder eine CH-Gruppierung steht.

Besonders hervorzuheben sind die Verbindungen der Formel (I), in welcher

A für Methan-1,1-diyl (Methylen, $-CH_2-$), Ethan-1,1-diyl

(Ethyliden, 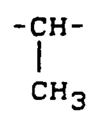 ),

Ethan-1,2-diyl (Dimethylen, $-CH_2CH_2-$), Propan-1,1-diyl

(Propyliden, 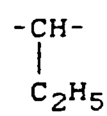 ),

Propan-1,2-diyl

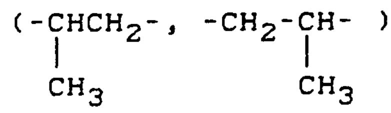

2-Methyl-propan-1,2-diyl

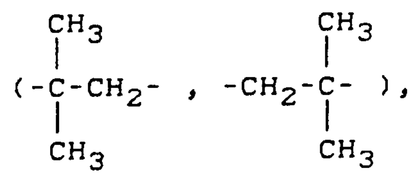

Propan-1,3-diyl (Trimethylen, $-CH_2CH_2CH_2-$), Butan-1,2-diyl

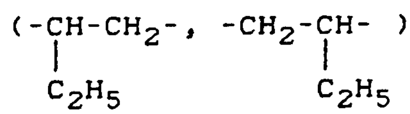

oder Butan-2,3-diyl

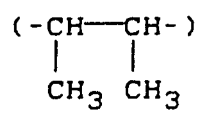

steht,

- $R^1$ für Hydroxy, Methoxy oder Ethoxy steht,
- $R^2$ für Wasserstoff, Fluor oder Methyl steht,
- $R^3$ für Wasserstoff oder Methyl steht,
- $R^4$ für Wasserstoff oder Methyl steht,
- $R^5$ für Wasserstoff oder Methyl steht,
- $R^6$ für Wasserstoff, Methyl oder Methoxy steht,
- $R^7$ für Methyl oder Methoxy steht,
- X für Sauerstoff steht,
- Y für Sauerstoff steht und
- Z für Stickstoff oder eine CH-Gruppierung steht.

Verwendet man für das erfindungsgemäße Herstellungsverfahren beispielsweise 2-Hydroxy-3-cyclohexen-1-carbonsäure-methylester und 2-Chlor-4,6-dimethoxy-s-triazin als Ausgangsstoffe, so kann der Reaktionsablauf durch das folgende Formelschema wiedergegeben werden:

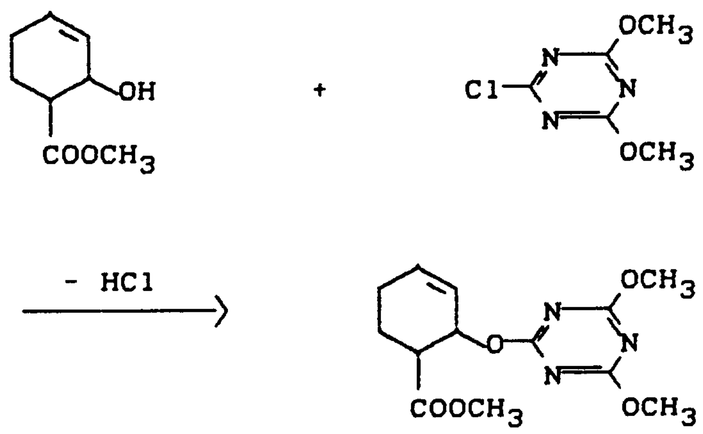


Die beim erfindungsgemäßen Verfahren zur Herstellung von Verbindungen der Formel (I) als Ausgangsstoffe zu verwendenden Cyclohexene sind durch die Formel (II) allgemein definiert.

In Formel (II) haben A, $R^1$, $R^2$, $R^3$, $R^4$, $R^5$, X und Y vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für A, $R^1$, $R^2$, $R^3$, $R^4$, $R^5$, X und Y angegeben wurden.

Beispiele für die Ausgangsstoffe der Formel (II) sind in der nachstehenden Tabelle 1 aufgeführt.

<u>Tabelle 1:</u> Beispiele für die Ausgangsstoffe der Formel (II)

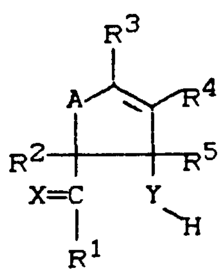

(II)

| A | $R^1$ | $R^2$ | $R^3$ | $R^4$ | $R^5$ | X | Y |
|---|---|---|---|---|---|---|---|
| $-CH_2-$ | OH | H | H | H | H | O | O |
| $-CH_2CH_2-$ | OH | H | H | H | H | O | O |

<u>Tabelle 1:</u> Beispiele für die Ausgangsstoffe der Formel (II) (Fortsetzung)

| A | $R^1$ | $R^2$ | $R^3$ | $R^4$ | $R^5$ | X | Y |
|---|---|---|---|---|---|---|---|
| 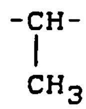 | OH | H | H | H | H | O | O |
| 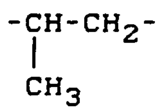 | OH | H | H | H | H | O | O |
| 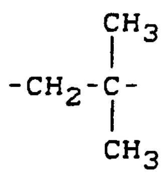 | OH | H | H | H | H | O | O |
| 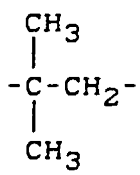 | $CH_3$ | H | H | H | $CH_3$ | O | O |
| 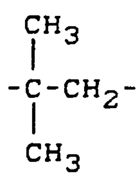 | $OC_2H_5$ | H | H | H | $CH_3$ | O | O |
| $-CH_2CH_2-$ | $OCH_3$ | H | H | H | H | O | O |
| $-CH_2CH_2-$ | $OCH_3$ | $CH_3$ | H | H | H | O | O |
| $-CH_2CH_2-$ | H | $CH_3$ | $CH_3$ | H | H | O | O |
| 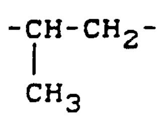 | $OCH_3$ | $CH_3$ | H | H | H | O | O |

Tabelle 1: Beispiele für die Ausgangsstoffe der Formel (II) (Fortsetzung)

| A | $R^1$ | $R^2$ | $R^3$ | $R^4$ | $R^5$ | X | Y |
|---|---|---|---|---|---|---|---|
| $-CH_2CH_2-$ | $OCH_3$ | H | $CH_3$ | H | H | O | O |
| $-CH(CH_3)-CH_2-$ | $OCH_3$ | H | H | H | H | O | NH |
| $-CH_2CH_2-$ | $OCH_3$ | H | H | H | H | O | NH |
| $-CH_2CH_2-$ | $OCH_3$ | $CH_3$ | H | H | H | O | NH |
| $-CH(CH_3)-CH_2-$ | $OC_2H_5$ | H | H | H | H | O | NH |
| $-CH(CH_3)-CH_2-$ | $OCH_3$ | H | H | H | H | O | NH |

Die Ausgangsstoffe der Formel (II) sind bekannt und/oder können nach an sich bekannten Verfahren hergestellt werden (vergleiche DE-OS 22 40 311; J. Org. Chem. 43 (1978), 3314-3319; loc. cit. 45 (1980), 2579-2581; J. Chem. Soc., Perkin I, 1981, 1096-1102; Chem. Pharm. Bull 34 (1986), 3488-3491; Tetrahedron Lett. 28 (1987), 4169-4172; EP-A 376 072).

Die beim erfindungsgemäßen Verfahren zur Herstellung von Verbindungen der Formel (I) weiter als Ausgangsstoffe zu verwendenden Azine sind durch die Formel (III) allgemein definiert.

In Formel (III) haben $R^6$, $R^7$ und Z vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^6$, $R^7$ und Z angegeben wurden, und

Q steht vorzugsweise für Fluor, Chlor, Brom oder $C_1$-$C_4$-Alkylsulfonyl, insbesondere für Chlor oder Methylsulfonyl.

Als Beispiele für die Ausgangsstoffe der Formel (III) seien genannt:

2-Chlor- 4,6-dimethyl-pyrimidin, 2-Chlor-4-methyl-6-methoxy-pyrimidin, 2-Chlor-4,6-dimethoxy-pyrimidin, 2-Chlor-4-methyl-6-ethoxy-pyrimidin, 2,4-Dichlor-6-methoxy-pyrimidin, 2-Chlor-methyl-pyrimidin, 2,4-Dichlor-6-methyl-pyrimidin, 2-Chlor-4-trifluormethyl-6-methoxy-pyrimidin, 2-Chlor-4-methoxy-6-difluormethoxy-pyrimidin, 2-Chlor-4-methyl-6-difluormethoxy-pyrimidin, 2-Chlor-4,6-bis-difluormethoxy-pyrimidin, 2,4-Dichlor-6-ethoxy-pyrimidin, 2-Chlor-4,6-diethoxy-pyrimidin, 2,4,5-Trichlor-6-methyl-pyrimidin, 2,5-Dichlor-4-methyl-6-methoxy-pyrimidin, 2,4,6-Trichlor-pyrimidin, 2-Chlor-4-ethyl-6-methoxy-pyrimidin, 2,5-Dichlor-4,6-dimethoxypyrimidin, 2-Chlor-4-methoxy-6-methylamino-pyrimidin und 2-Chlor-4,6-bis-trifluormethyl-pyrimidin, 2-Chlor-4,6-dimethyl-s-triazin, 2-Chlor-4-methoxy-6-methyl-s-triazin, 2-Chlor-4,6-dimethoxy-s-triazin, 2-Chlor-4-ethoxy-6-methyl-s-triazin und 2-Chlor-4-ethyl-6-methoxy-s-triazin, sowie 2-Methylsulfonyl-4,6-dimethyl-pyrimidin, 2-Methylsulfonyl-4-methyl-6-methoxy-pyrimidin, 2-Methylsulfonyl-4,6-dimethoxy-pyrimidin, 2-Methylsulfonyl-4-methyl-6-ethoxypyrimidin, 2-Methylsufonyl-4-chlor-6-methoxy-pyrimidin, 2-Methylsulfonyl-4-methyl-pyrimidin, 2-Methylsulfonyl-4-chlor-6-methyl-pyrimidin, 2-Methylsulfonyl-4-trifluormethyl-6-methoxy-pyrimidin, 2-Methylsulfonyl-4-methoxy-6-difluormethoxy-pyrimidin, 2-Methylsulfonyl-4-methyl-6-difluormethoxy-pyrimidin, 2-Methylsulfonyl-4,6-bis-difluormethoxy-pyrimidin, 2-Methylsulfonyl-4-chlor-6-ethoxy-pyrimidin, 2-Methylsulfonyl-4,6-diethoxy-pyrimidin, 2-Methylsulfonyl-4,5-dichlor-6-methyl-pyrimidin, 2-Methylsulfonyl-4-methyl-5-chlor-6-methoxy-pyrimidin, 2-Methylsulfonyl-4,6-dichlor-pyrimidin, 2-Methylsulfonyl-4-ethyl-6-methoxy-pyrimidin, 2-Methylsulfonyl-5-chlor-4,6-dimethoxy-pyrimidin, 2-Methylsulfonyl-4-methoxy-6-methylamino-pyrimidin und 2-Methylsulfonyl-4,6-bis-trifluormethyl-pyrimidin.

Die Azine der Formel (III) sind bekannt und/oder können nach an sich bekannten Verfahren hergestellt werden (vgl. J. Chem. Soc. 1957, 1830, 1833; J. Org. Chem. 26 (1961), 792; US-P 3308119; US-P 4711959).

Das erfindungsgemäße Verfahren zur Herstellung der neuen Verbindungen der Formel (I) wird vorzugsweise unter Verwendung von Verdünnungsmitteln durchgeführt. Als Verdünnungsmittel kommen dabei inerte organische Lösungsmittel infrage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutylketon, Ester wie Essigsäuremethylester und -ethylester, Nitrile wie z.B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Als Säureakzeptoren können bei dem erfindungsgemäßen Verfahren alle üblicherweise für derartige Umsetzungen verwendbaren Säurebindemittel eingesetzt werden. Vorzugsweise infrage kommen Alkalimetallhydroxide, wie z.B. Natrium- und Kaliumhydroxid, Erdalkalihydroxide wie z.B. Calciumhydroxid, Alkalicarbonate und -alkoholate wie Natrium- und Kaliumcarbonat, Natrium- und Kalium-tert-Butylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo[5,4,0]-undec-7-en (DBU) und 1,4-Diazabicyclo-[2,2,2]-octan (DABCO).

Die Reaktionstemperaturen können bei dem erfindungsgemäßen Verfahren in einem größeren Bereich variiert werden. Im allgemeinen arbeitet man bei Temperaturen zwischen 0 °C und 150 °C, vorzugsweise bei Temperaturen zwischen 10 °C und 100 °C.

Das erfindungsgemäße Verfahren wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren jeweils nach üblichen Methoden (vergleiche die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich zur Bekämpfung von monokotylen und dikotylen Unkräutern sowohl im Vorauflauf- als auch im Nachauflauf-Verfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen infrage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D, 2,4 DB, 2,4 DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate,

Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 10 g und 10 kg Wirkstoff pro Hektar Bodenfläche, vorzugsweise zwischen 50 g und 5 kg pro ha.

Die Herstellung und die Verwendung der erfindungsgemäßen Wirkstoffe geht aus den nachfolgenden Beispielen hervor.

Herstellungsbeispiele:

Beispiel 1

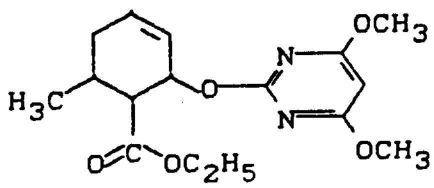

Eine Mischung aus 1,2 g (5,5 mMol) 4,6-Dimethoxy-2-methylsulfonyl-pyrimidin, 1,0 g (8,2 mMol) 3-Hydroxy-5-methylcyclohexen-4-carbonsäureethylester, 1,1 g (5,5 mMol) Kaliumcarbonat und 20 ml Acetonitril wird 12 Stunden unter Rückfluß erhitzt und anschließend eingeengt. Der Rückstand wird mit Wasser / Essigsäureethylester ausgeschüttelt, die organische Phase abgetrennt, mit Natriumsulfat getrocknet und filtriert. Vom Filtrat wird das Lösungsmittel im Wasserstrahlvakuum abdestilliert.

Man erhält 1,2 g (71 % der Theorie) 3-(4,6-Dimethoxypyrimdin-2-yloxy)-5-methylcyclohexen-4-carbonsäureethylester als öligen Rückstand.

Analog Beispiel 1 und entsprechend der allgemeinen Beschreibung des erfindungsgemäßen Herstellungsverfahrens können beispielsweise auch die in der nachstehenden Tabelle 2 aufgeführten Verbindungen der Formel (I) hergestellt werden:

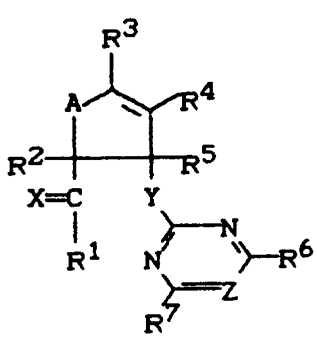

(I)

Tabelle 2: Beispiele für die Verbindungen der Formel (I)

| Bsp.-Nr. | A | $R^1$ | $R^2$ | $R^3$ | $R^4$ | $R^5$ | $R^6$ | $R^7$ | X | Y | Z | Physikal. Daten |
|---|---|---|---|---|---|---|---|---|---|---|---|---|
| 2 | $-CHCH_2-$<br>$CH_3$ | OH | H | H | H | H | $OCH_3$ | $OCH_3$ | O | O | CH | (Öl) |
| 3 | $-CH-CH_2-$<br>$C_6H_5$ | OH | H | H | H | H | $OCH_3$ | $OCH_3$ | O | O | CH | (Öl) |
| 4 | $-CH-CH_2-$<br>$COOCH_3$ | $OCH_3$ | H | H | H | H | $OCH_3$ | $OCH_3$ | O | O | CH | (Öl) |
| 5 | $-CH_2-CH_2-$ | $OCH_3$ | H | H | H | H | $OCH_3$ | $OCH_3$ | O | O | CH | (Öl) |
| 6 | $-CH_2-CH_2-$ | OH | H | H | H | H | $OCH_3$ | $OCH_3$ | O | O | CH | (Öl) |
| 7 | $-CH_2-CH_2-$ | $OC_2H_5$ | H | H | H | H | $OCH_3$ | $OCH_3$ | O | O | CH | (Öl) |
| 8 | $-CH_2-CH_2-$ | $OCH_2$ 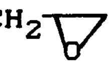 | H | H | H | H | $OCH_3$ | $OCH_3$ | O | O | CH | (Öl) |
| 9 | $-CH_2-CH_2-$ | $OC(CH_3)_3$ | H | H | H | H | $OCH_3$ | $OCH_3$ | O | O | CH | (Öl) |
| 10 | $-CH_2-CH_2-$ | $OC_2H_5$ | $CH_3$ | H | H | H | $OCH_3$ | $OCH_3$ | O | O | CH | Fp: 65° C |
| 11 | $-CH_2-CH_2-$ | OH | $CH_3$ | H | H | H | $OCH_3$ | $OCH_3$ | O | O | CH | (Öl) |

Tabelle 2: (Fortsetzung)

| Bsp.-Nr. | A | $R^1$ | $R^2$ | $R^3$ | $R^4$ | $R^5$ | $R^6$ | $R^7$ | X | Y | Z | Physikal. Daten |
|---|---|---|---|---|---|---|---|---|---|---|---|---|
| 12 | $-CH_2-CH_2-$ | $OC_4H_9$ | F | H | H | H | $OCH_3$ | $OCH_3$ | O | O | CH | (Öl) |
| 13 | $-CH_2-CH_2-$ | OH | F | H | H | H | $OCH_3$ | $OCH_3$ | O | O | CH | Fp: 145° C |
| 14 | $-CH_2-CH_2-$ | $OCH_3$ | $-CH_2COOCH_3$ | H | H | H | $OCH_3$ | $OCH_3$ | O | O | CH | Fp: 95° C |
| 15 | $-CH_2-CH_2-$ | OH | $-CH_2COOCH_3$ | H | H | H | $OCH_3$ | $OCH_3$ | O | O | CH | (Öl) |
| 16 | $-CH-CH_2$<br>\|<br>$CH_3$ | $OC_2H_5$ | H | H | H | H | Cl | $OCH_3$ | O | O | CH | (Öl) |
| 17 | $-CH-CH_2$<br>\|<br>$CH_3$ | OH | H | H | H | H | Cl | $OCH_3$ | O | O | CH | (Öl) |
| 18 | $-CH_2-CH_2$ | $OC_2H_5$ | H | H | H | H | Cl | $OCH_3$ | O | O | CH | (Öl) |
| 19 | $-CH_2-CH_2-$ | OH | H | H | H | H | Cl | $OCH_3$ | O | O | CH | (Öl) |
| 20 | $-CH_2-CH_2-$ | $OCH_3$ | $CF_3$ | H | H | H | $OCH_3$ | $OCH_3$ | O | O | CH | (Öl) |
| 21 | $-CH-CH_2-$<br>\|<br>$CH_3$ | $OC_2H_5$ | H | H | H | H | $OCH_3$ | $OCH_3$ | O | O | CH | (Öl) |

Ausgangsstoffe der Formel (II):

Beispiel (II-1)

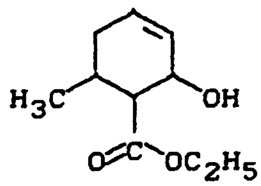

5,0 g (27,4 mMol) 5-Methyl-3-oxocyclohexen-4-carbonsäureethylester werden in 40 ml Methanol vorgelegt, auf 0°C abgekühlt und mit 10,2 g (27,4 mMol) Cer(III)-chlorid-Heptahydrat und 1,1 g (27,4 mMol) Natriumborhydrid versetzt. Nach zweistündigem Rühren bei Raumtemperatur wird es in Wasser gegossen und mit Dichlormethan extrahiert. Die vereinigten organischen Phasen werden mit Natriumsulfat getrocknet und filtriert. Das Filtrat wird eingeengt.

Man erhält 4,6 g (92 % der Theorie) 3-Hydroxy-5-methylcyclohexen-4-carbonsäureethylester als öligen Rückstand.

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flacheneinheit. Nach drei Wochen wird der Schädigungsgrad der Pflanzen bonitiert in % Schädigung im Vergleich zur Entwicklung der unbehandelten Kontrolle. Es bedeuten:
0 % = keine Wirkung (wie unbehandelte Kontrolle)
100 % = totale Vernichtung
In diesem Test zeigen die Verbindungen gemäß den Herstellungsbeispielen 1 und 2 sehr starke Wirkung gegen Unkräuter.

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben, so, daß die jeweils gewünschten Wirkstoffmengen pro Flächeneinheit ausgebracht werden. Die Konzentration der Spritzbrühe wird so gewählt, daS in 1000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:
0 % = keine Wirkung (wie unbehandelte Kontrolle)
100 % = totale Vernichtung
In diesem Test zeigen die Verbindungen gemäß den Herstellungsbeispielen 1 und 2 sehr starke Wirkung

gegen Unkräuter.

**Patentansprüche**

1. Substituierte Cycloalkene der allgemeinen Formel (I)

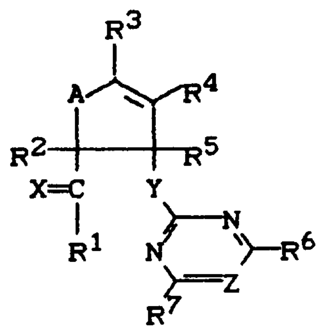

(I)

dadurch gekennzeichnet, daß

| | |
|---|---|
| A | für geradkettiges oder verzweigtes, gegebenenfalls substituiertes Alkandiyl steht, |
| $R^1$ | für Wasserstoff, Hydroxy, Amino oder für einen jeweils gegebenenfalls substituierten Rest der Reihe Alkyl, Alkoxy, Alkylthio, Alkylamino oder Dialkylamino steht, |
| $R^2$, $R^3$, $R^4$ und $R^5$ | gleich oder verschieden sind und für Wasserstoff, Hydroxy, Amino, Cyano, Halogen, Alkyl, Halogenalkyl, Alkoxy, Alkylthio, Aryl oder Alkoxycarbonyl oder Alkoxycarbonylalkyl stehen, |
| $R^6$ und $R^7$ | gleich oder verschieden sind und für Wasserstoff, Halogen oder für einen jeweils gegebenenfalls substituierten Rest der Reihe Alkyl, Alkoxy, Alkylthio, Alkylamino oder Dialkylamino stehen, |
| X | für Sauerstoff, Schwefel oder eine der nachstehenden Gruppierungen steht |
| N-$R^8$ | oder |

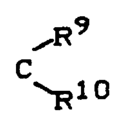

wobei

| | |
|---|---|
| $R^8$ | für Wasserstoff, Hydroxy, Amino oder füreinen jeweils gegebenenfalls substituierten Rest der Reihe Alkyl, Alkenyl, Alkinyl, Alkoxy, Alkenyloxy, Alkoxycarbonylalkoxy, Alkylamino, Dialkylamino, Alkylcarbonylamino, Alkoxycarbonylamino, Alkylsulfonylamino, Aryl, Aralkyl, Aryloxy, Aralkyloxy, Arylamino, Aralkylamino, N-Alkyl-N-arylamino, Hetarylamino, Hetarylcarbonylamino, Arylcarbonylamino oder Arylsulfonylamino steht, |
| $R^9$ | für Wasserstoff, Halogen, Cyano, Carboxyl, Alkoxycarbonyl, Alkylcarbonylamino oder Dialkoxyphosphoryl steht und |
| $R^{10}$ | für Formyl, Cyano, Carboxyl, Hydroxymethyl, Carbamoyl oder für einen jeweils gegebenenfalls substituierten Rest der Reihe Alkoxycarbonyl, Cycloalkoxycarbonyl, Alkylthiocarbonyl, Alkylaminocarbonyl, Cycloalkylaminocarbonyl, Dialkylaminocarbonyl, Alkylaminocarbonylalkoxycarbonyl, Dialkylaminocarbonylalkoxycarbonyl, Arylaminocarbonylalkoxycarbonyl, N-Alkyl-N-arylaminocarbonylalkoxycarbonyl, Pyrrolidinylcarbonyl, Piperidinylcarbonyl, Morpholinylcarbonyl, Piperazinylcarbonyl, Aryloxycarbonyl, Aralkyloxycarbonyl, Heterocyclylalkoxycarbonyl, Arylthiocarbonyl, Aralkylthiocarbonyl, Arylaminocarbonyl, Aralkylaminocarbonyl, N-Alkyl-N-arylaminocarbonyl, Arylhydrazinocarbonyl, Al- |

kylhydrazinocarbonyl, Phthalimidoxycarbonyl oder $R^{10}$ zusammen mit $R^9$ für die Gruppierung $-CO-O-(CH_2)_n$-steht,wobei n für die Zahlen 1 bis 4 steht,

Y für Sauerstoff, Schwefel, Imino (NH) oder Alkylimino (N-Alkyl) steht, und

Z für Stickstoff oder die Gruppierung $C-R^{11}$ steht, worin $R^{11}$ für Wasserstoff, Halogen, Alkyl oder Alkoxy steht.

**2.** Substituierte Cycloalkene der allgemeinen Formel (I) gemäß Anspruch 1, dadurch gekennzeichnet, daß

A für geradkettiges oder verzweigtes Alkandiyl mit 1 bis 5 Kohlenstoffatomen steht, welches gegebenenfalls durch Hydroxy, Amino, Halogen, Phenyl oder $C_1$-$C_5$-Alkoxycarbonyl substituiert ist,

$R^1$ für Wasserstoff, Hydroxy, Amino oder für einen jeweils gegebenenfalls durch Halogen, $C_1$-$C_5$-Alkoxy oder Oxiranyl substituierten Rest der Reihe $C_1$-$C_5$-Alkyl, $C_1$-$C_5$-Alkoxy, $C_1$-$C_5$-Alkylthio, $C_1$-$C_5$-Alkylamino oder Di-($C_1$-$C_5$-alkyl)-amino steht,

$R^2$, $R^3$, $R^4$ und $R^5$ gleich oder verschieden sind und für Wasserstoff, Hydroxy, Amino, Cyano, Halogen, $C_1$-$C_5$-Alkyl, $C_1$-$C_5$-Alkoxy, $C_1$-$C_5$-Alkylthio, Phenyl oder $C_1$-$C_5$-Alkoxycarbonyl stehen,

$R^6$ und $R^7$ gleich oder verschieden sind und für Wasserstoff, Halogen oder für einen jeweils gegebenenfalls durch Halogen oder $C_1$-$C_3$-Alkoxy substituierten Rest der Reihe $C_1$-$C_4$-Alkyl, $C_1$-$C_4$-Alkoxy, $C_1$-$C_4$-Alkylthio, $C_1$-$C_4$-Alkylamino oder Di-($C_1$-$C_4$-alkyl)-amino stehen,

X für Sauerstoff, Schwefel oder eine der nachstehenden Gruppierungen steht

$N-R^8$ oder

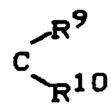

wobei

$R^8$ für Wasserstoff, Hydroxy, Amino oder für einen jeweils gegebenenfalls durch Halogen substituierten Rest der Reihe $C_1$-$C_6$-Alkyl, $C_3$-$C_6$-Alkenyl, $C_3$-$C_6$-Alkinyl, $C_1$-$C_6$-Alkoxy, $C_3$-$C_6$-Alkenyloxy, $C_1$-$C_4$-Alkoxy-carbonyl-$C_1$-$C_2$-alkoxy, $C_1$-$C_6$-Alkylamino, Di-($C_1$-$C_2$-alkyl)-amino, $C_1$-$C_6$-Alkylcarbonylamino, $C_1$-$C_6$-Alkoxycarbonylamino, $C_1$-$C_6$-Alkyl-sulfonylamino, oder für einen jeweils gegebenenfalls durch Nitro, Amino, Cyano, Carboxyl, Halogen, $C_1$-$C_4$-Alkyl, $C_1$-$C_2$-Halogenalkyl, $C_1$-$C_4$-Alkoxy, $C_1$-$C_2$-Halogenalkoxy, $C_1$-$C_4$-Alkylthio, $C_1$-$C_2$-Halogenalkylthio, $C_1$-$C_4$-Alkoxy-carbonyl und/ oder Di-($C_1$-$C_2$-alkyl)-amino substituierten Rest der Reihe Phenyl, Phenyl-$C_1$-$C_4$-alkyl, Phenoxy, Phenyl-$C_1$-$C_4$-alkoxy, Phenylamino, Phenyl-$C_1$-$C_4$-alkylamino, N-($C_1$-$C_4$-alkyl)-N-phenylamino, Pyridylamino, Pyrimidylamino, Pyridylcarbonylamino, Phenylcarbonylamino, Furylcarbonylamino, Thienylcarbonylamino oder Phenylsulfonylamino steht,

$R^9$ für Wasserstoff, Halogen, Cyano, Carboxyl, $C_1$-$C_6$-Alkoxycarbonyl, $C_1$-$C_6$-Alkylcarbonylamino oder Di-($C_1$-$C_4$-alkoxy)-phosphoryl steht und

$R^{10}$ für Formyl, Cyano, Carboxyl, Hydroxymethyl, Carbamoyl oder für einen jeweils gegebenenfalls durch Halogen, Carboxyl oder $C_1$-$C_4$-Alkoxycarbonyl substituierten Rest der Reihe $C_1$-$C_6$-Alkoxycarbonyl, $C_5$-$C_6$-Cycloalkyloxycarbonyl, $C_1$-$C_6$-Alkylthiocarbonyl, Alkylaminocarbonyl, oder $C_5$-$C_6$-Cycloalkylaminocarbonyl,Di-($C_1$-$C_2$-alkyl)-aminocarbonyl, $C_1$-$C_4$-Alkylaminocarbonyl-$C_1$-$C_4$-alkoxycarbonyl, Di-($C_1$-$C_2$-alkyl)-aminocarbonyl-$C_1$-$C_4$-alkoxycarbonyl, Phenylaminocarbonyl-$C_1$-$C_4$-alkoxycarbonyl, N-Methyl-N-phenylaminocarbonyl-$C_1$-$C_4$-alkoxycarbonyl, oder für einen jeweils gegebenenfalls durch Methyl und/oder Ethyl substituierten Rest der Reihe Pyrrolidinylcarbonyl, Piperidinylcarbonyl, Morpholinylcarbonyl oder Piperazinylcarbonyl,

für jeweils gegebenenfalls durch Nitro, Amino, Cyano, Carboxyl, Halogen, $C_1$-

$C_4$-Alkyl, $C_1$-$C_2$-Halogenalkyl, $C_1$-$C_4$-Alkoxy, $C_1$-$C_2$-Halogenalkoxy, $C_1$-$C_4$-Alkylthio, $C_1$-$C_2$-Halogenalkylthio, $C_1$-$C_4$-Alkoxy-carbonyl und/oder Di-($C_1$-$C_2$-alkyl)-amino substituierten Rest der Reihe Phenoxycarbonyl, Phenyl-$C_1$-$C_4$-alkoxycarbonyl, Furylmethoxycarbonyl, Thienylmethoxycarbonyl, Phenylthiocarbonyl, Phenyl-$C_1$-$C_4$-alkylthiocarbonyl, Phenylaminocarbonyl, Phenyl-$C_1$-$C_4$-alkylaminocarbonyl,N-($C_1$-$C_4$-Alkyl)-N-phenylaminocarbonyl oder Phenylhydrazinocarbonyl, $C_1$-$C_4$-Alkylhydrazinocarbonyl, für Phthalimidoxycarbonyl oder zusammen mit $R^9$ -CO-O-$(CH_2)_n$-steht, wobei

n für die Zahlen 1 bis 4 steht,

Y für Sauerstoff, Schwefel, Imino (NH) oder Methylimino ($NCH_3$) steht und

Z für Stickstoff oder die Gruppierung C-$R^{11}$ steht,

worin

$R^{11}$ für Wasserstoff, Fluor, Chlor, Methyl oder Methoxy steht.

**3.** Substituente Cycloalkene der allgemeinen Formel (I) gemäß Anspruch 1 dadurch gekennzeichnet, daß

A für Methan-1,1-diyl (Methylen, $-CH_2-$), Ethan-1,1-diyl

(Ethyliden, 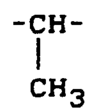 ),

Ethan-1,2-diyl (Dimethylen, $-CH_2CH_2-$), Propan-1,1-diyl

(Propyliden, 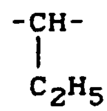 ),

Propan-1,2-diyl

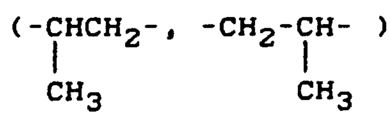

2-Methyl-propan-1,2-diyl

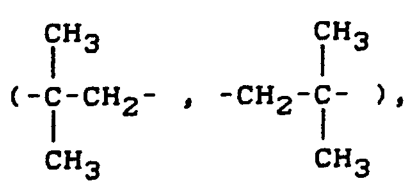

Propan-1,3-diyl (Trimethylen, $-CH_2CH_2CH_2-$), Butan-1,2-diyl

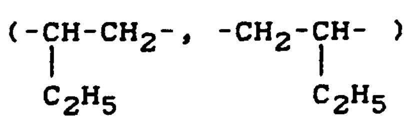

oder Butan-2,3-diyl

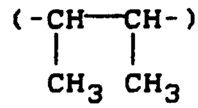

steht,

$R^1$ für Wasserstoff, Hydroxy, Amino oder für einen jeweils gegebenenfalls durch Fluor, Chlor, Methoxy oder Ethoxy substituierten Rest der Reihe Methyl, Ethyl, Propyl, Isopropyl, Methoxy, Oxiranylmethoxy, Ethoxy, Propoxy, Isopropoxy, Methylamino, Ethylamino, Propylamino, Isopropylamino, Dimethylamino oder Diethylamino steht,

$R^2$, $R^3$, $R^4$ und $R^5$ gleich oder verschieden sind und für Wasserstoff, Hydroxy, Amino, Fluor, Chlor, Brom, Methyl, Ethyl, Propyl, Isopropyl, Phenyl, Methoxycarbonyl oder Ethoxycarbonyl stehen,

$R^6$ und $R^7$ gleich oder verschieden sind und für Wasserstoff, Chlor, Methyl, Ethyl, Trifluormethyl, Methoxymethyl, Methoxy, Ethoxy, Difluormethoxy, Methylthio, Methylamino, Ethylamino oder Dimethylamino stehen,

X für Sauerstoff, Schwefel oder eine der nachstehenden Gruppierungen steht

$N-R^8$ oder

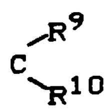

wobei

$R^8$ für Wasserstoff, Hydroxy, Amino, für Methyl, Ethyl, n-Propyl, Isopropyl, n-Butyl, Isobutyl, sec-Butyl, tert-Butyl, Allyl, Propargyl, Methoxy, Ethoxy, n-Propoxy, Isopropoxy, n-Butoxy, Isobutoxy, sec-Butoxy, Allyloxy, Methoxycarbonylmethoxy, Ethoxycarbonylmethoxy, Methoxycarbonylethoxy, Ethoxycarbonylethoxy, Methylamino, Ethylamino, n-Propylamino, Isopropylamino, n-Butylamino, Isobutylamino, sec-Butylamino, tert-Butylamino, Dimethylamino, Acetylamino, Propionylamino, Methoxycarbonylamino, Ethoxycarbonylamino, Methylsulfonylamino, Ethylsulfonylamino, für jeweils gegebenenfalls durch Nitro, Cyano, Fluor, Chlor, Brom, Methyl, Trifluormethyl, Methoxy, Trifluormethoxy, Methylthio oder Trifluormethylthio substituiertes Phenyl, Benzyl, Phenoxy, Benzyloxy, Phenylamino, Benzylamino, N-Methyl-N-phenylamino, Pyridylamino, Pyrimidylamino, Pyridylcarbonylamino, Phenylcarbonylamino, Furylcarbonylamino, Thienylcarbonylamino oder Phenylsulfonylamino steht,

$R^9$ für Wasserstoff, Fluor, Chlor, Cyano, Carbo-xyl, $C_1$-$C_4$-Alkoxycarbonyl, $C_1$-$C_4$-Alkyl-carbonyl-amino, Dimethoxyphosphoryl oder Diethoxyphosphoryl steht und

$R^{10}$ für Formyl, Cyano, Carboxyl, Hydroxymethyl, Carbamoyl, für einen jeweils gegebenenfalls durch Fluor, Chlor, Carboxy oder $C_1$-$C_4$-Alkoxycarbonyl substituierten Rest der Reihe $C_1$-$C_4$-Alkoxycarbonyl, $C_5$-$C_6$-Cycloalkyloxycarbonyl, $C_1$-$C_4$-Alkylthiocarbonyl, $C_1$-$C_4$-Alkylaminocarbonyl, $C_5$-$C_6$-Cycloalkylaminocarbonyl, Dimethylaminocarbonyl, für $C_1$-$C_4$-Alkylaminocarbonyl-$C_1$-$C_4$-alkoxycarbonyl, Dimethylaminocarbonyl-$C_1$-$C_4$-alkoxycarbonyl, N-Methyl-N-phenylaminocarbonyl-$C_1$-$C_4$-alkoxycarbonyl, oder einen jeweils gegebenenfalls durch Methyl und/oder Ethyl substituierten Rest der Reihe Pyrrolidinylcarbonyl, Piperidinylcarbonyl, Morpholinylcarbonyl oder Piperazinylcarbonyl,

oder für gegebenenfalls durch Nitro, Cyano, Fluor, Chlor, Brom, Methyl, Trifluormethyl, Methoxy, Trifluormethoxy, Methylthio oder Trifluormethylthio substituierten Rest der Reihe Phenoxycarbonyl, Benzyloxycarbonyl, Phenylthiocarbonyl, Benzylthiocarbonyl, Phenylaminocarbonyl, Benzylaminocarbonyl, N-Methyl-N-phenylaminocarbonyl, Phenylhydrazinocarbonyl, für Phthalimidoxycarbonyl oder zusammen mit $R^9$ für die Gruppierungen $-CO-O-CH_2CH_2-$, $-COO-CH_2CH_2CH_2-$,

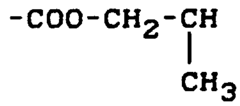

oder

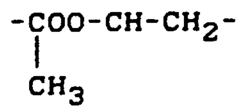

steht,

Y für Sauerstoff oder Imino (NH) steht und

Z für Stickstoff oder eine CH-Gruppierung steht.

**4.** Substitierte Cycloalkene der allgemeinen Formel (I) gemäß Anspruch 1, dadurch gekennzeichnet, daß

A für Methan-1,1-diyl (Methylen, $-CH_2-$), Ethan-1,1-diyl

(Ethyliden, 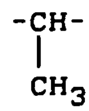 ),

Ethan-1,2-diyl (Dimethylen, $-CH_2CH_2-$), Propan-1,1-diyl

(Propyliden, 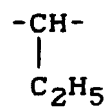 ),

Propan-1,2-diyl

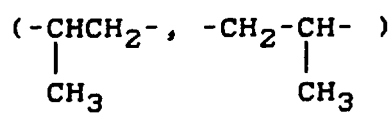

2-Methyl-propan-1,2-diyl

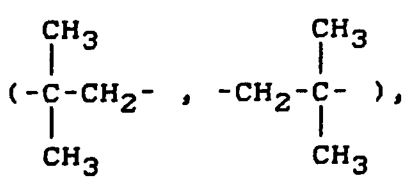

Propan-1,3-diyl (Trimethylen, $-CH_2CH_2CH_2-$), Butan-1,2-diyl

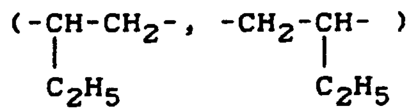

oder Butan-2,3-diyl

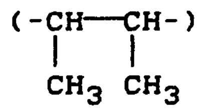

steht,

| | |
|---|---|
| $R^1$ | für Hydroxy, Methoxy oder Ethoxy steht, |
| $R^2$ | für Wasserstoff, Fluor oder Methyl steht, |
| $R^3$ | für Wasserstoff oder Methyl steht, |
| $R^4$ | für Wasserstoff oder Methyl steht, |
| $R^5$ | für Wasserstoff oder Methyl steht, |
| $R^6$ | für Wasserstoff, Methyl oder Methoxy steht, |
| $R^7$ | für Methyl oder Methoxy steht, |
| X | für Sauerstoff steht, |
| Y | für Sauerstoff steht und |
| Z | für Stickstoff oder eine CH-Gruppierung steht. |

**5.** Verfahren zur Herstellung von substituierten Cycloalkenen der allgemeinen Formel (I)

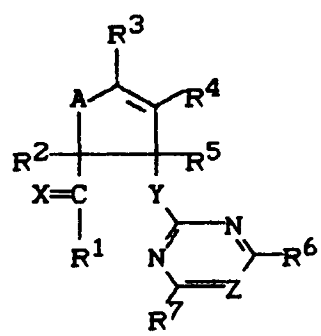

(I)

in welcher

| | |
|---|---|
| A | für geradkettiges oder verzweigtes, gegebenenfalls substituiertes Alkandiyl steht, |
| $R^1$ | für Wasserstoff, Hydroxy, Amino oder für einen jeweils gegebenenfalls substituierten Rest der Reihe Alkyl, Alkoxy, Alkylthio, Alkylamino oder Dialkylamino steht, |
| $R^2$, $R^3$, $R^4$ und $R^5$ | gleich oder verschieden sind und für Wasserstoff, Hydroxy, Amino, Cyano, Halogen, Alkyl, Alkoxy, Alkylthio, Aryl oder Alkoxycarbonyl stehen, |
| $R^6$ und $R^7$ | gleich oder verschieden sind und für Wasserstoff, Halogen oder für einen jeweils gegebenenfalls substituierten Rest der Reihe Alkyl, Alkoxy, Alkylthio, Alkylamino oder Dialkylamino stehen, |
| X | für Sauerstoff, Schwefel oder eine der nachstehenden Gruppierungen steht |
| N-$R^8$ | oder |

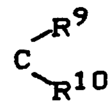

wobei

$R^8$ für Wasserstoff, Hydroxy, Amino oder für einen jeweils gegebenenfalls substituierten Rest der Reihe Alkyl, Alkenyl, Alkinyl, Alkoxy, Alkenyloxy, Alkoxycarbonylalkoxy, Alkylamino, Dialkylamino, Alkylcarbonylamino, Alkoxycarbonylamino, Alkylsulfonylamino, Aryl, Aralkyl, Aryloxy, Aralkyloxy, Arylamino, Aralkylamino, N-Alkyl-N-arylamino, Hetarylamino, Hetarylcarbonylamino, Arylcarbonylamino oder Arylsulfonylamino steht,

$R^9$ für Wasserstoff, Halogen, Cyanol, Carboxy, Alk oxycarbonyl, Alkylcarbonylamino oder Dialkoxyphosphoryl steht und

$R^{10}$ für Formyl, Cyano, Carboxyl, Hydroxymethyl, Carbamoyl oder für einen jeweils gegebenenfalls substituierten Rest der Reihe Alkoxycarbonyl, Cycloalkoxycarbonyl, Alkylthiocarbonyl, Alkylaminocarbonyl, Cycloalkylaminocarbonyl, Dialkylaminocarbonyl, Alkylaminocarbonylalkoxycarbonyl, Dialkylaminocarbonylalkoxycarbonyl, Arylaminocarbonylalkoxycabonyl, N-Alkyl-N-arylamino carbonylalkoxycarbonyl, Pyrrolidinylcarbonyl, Piperidinylcarbonyl, Morpholinylcarbonyl, Piperazinylcarbonyl, Aryloxycarbonyl, Aralkyloxycarbonyl, Heterocyclylalkoxycarbonyl, Arylthiocarbonyl, Aralkylthiocarbonyl, Arylaminocarbonyl, Aralkylaminocarbonyl, N-Alky N-arylaminocarbonyl, Arylhydrazinocarbonyl, Alkylhydrazinocarbonyl, Phthalimidoxycarbonyl oder $R^{10}$ zusammen mit $R^9$ für die Gruppierung $-CO-O-(CH_2)_n$-steht, wobei n für die Zahlen 1 bis 4 steht,

Y für Sauerstoff, Schwefel, Imino (NH) oder Alkylimino (N-Alkyl) steht, und

Z für Stickstoff oder die Gruppierung $C-R^{11}$ steht, worin $R^{11}$ für Wasserstoff, Halogen, Alkyl oder Alkoxy steht,

dadurch gekennzeichnet, daß man Cycloalkene der allgemeinen Formel (II)

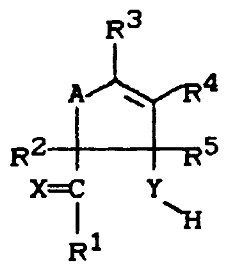

(II)

in welcher

A, $R^1$, $R^2$, $R^3$, $R^4$, $R^5$, X und Y die oben angegebenen Bedeutungen haben,

mit Azinen der allgemeinen Formel (III)

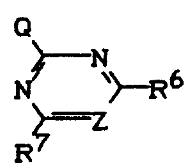

(III)

in welcher

$R^6$, $R^7$ und Z die oben angegebenen Bedeutungen haben und

Q für eine nucleofuge Abgangsgruppe steht,

gegebenenfalls in Gegenwart eines Säureakzeptors und gegebenenfalls in Gegenwart eines Verdünnungsmittels umsetzt.

**6.** Herbizide Mittel, gekennzeichnet durch einen Gehalt an mindestens einem substituierten Cycloalken der Formel (I) gemäß Anspruch 1 oder 5.

**7.** Verfahren zur Bekämpfung von unerwünschten Pflanzen, dadurch gekennzeichnet, daß man substituierte Cycloalkene der Formel (I) gemäß Anspruch 1 oder 5 auf die unerwünschten Pflanzen und/oder ihren Lebensraum einwirken läßt.

**8.** Verwendung von substiuierten Cycloalkenen der Formel (I) gemäß Anspruch 1 oder 5 zur Bekämpfung von unerwünschten Pflanzen.

**9.** Verfahren zur Herstellung von herbiziden Mitteln, dadurch gekennzeichnet, daß man substituierte Cycloalkene gemäß Anspruch 1 oder 5 mit Streckmitteln und/oder oberflächenaktiven Substanzen vermischt.

Europäisches Patentamt

# EUROPÄISCHER RECHERCHENBERICHT

Nummer der Anmeldung

EP 92 11 4571

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A | US-A-4 973 354 (MASATALTA HATANAKA ET AL.)<br>* das ganze Dokument *<br>----- | 1,6-9 | C07D239/60<br>C07D239/52<br>A01N43/54 |
| | | | **RECHERCHIERTE SACHGEBIETE (Int. Cl.5 )** |
| | | | C07D<br>A01N |

Der vorliegende Recherchenbericht wurde für alle Patentansprüche erstellt

| Recherchenort | Abschlußdatum der Recherche | Prüfer |
|---|---|---|
| DEN HAAG | 30 NOVEMBER 1992 | FRANCOIS J.C. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)